(19) Europäisches Patentamt / European Patent Office / Office européen des brevets

(11) **EP 4 017 447 B1**

(12) **FASCICULE DE BREVET EUROPEEN**

(45) Date de publication et mention de la délivrance du brevet:
**22.11.2023 Bulletin 2023/47**

(21) Numéro de dépôt: **20775912.7**

(22) Date de dépôt: **19.08.2020**

(51) Classification Internationale des Brevets (IPC):
*A61F 13/08* (2006.01)      *D04B 21/18* (2006.01)

(52) Classification Coopérative des Brevets (CPC):
**A61F 13/08; D04B 21/18;** D10B 2403/021;
D10B 2403/0311; D10B 2509/028

(86) Numéro de dépôt international:
**PCT/FR2020/051485**

(87) Numéro de publication internationale:
**WO 2021/032931 (25.02.2021 Gazette 2021/08)**

(54) **BANDE DE CONTENTION A SURFACE OPTIMISÉE**

KOMPRESSIONSBANDAGE MIT OPTIMIERTER OBERFLÄCHE

COMPRESSION BANDAGE WITH OPTIMIZED SURFACE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.08.2019 FR 1909345**

(43) Date de publication de la demande:
**29.06.2022 Bulletin 2022/26**

(73) Titulaire: **URGO RECHERCHE INNOVATION ET DEVELOPPEMENT**
**21300 Chenôve (FR)**

(72) Inventeurs:
• **COHADE, Céline**
**42650 SAINT-JEAN-BONNEFONDS (FR)**
• **GRANGE, David**
**42210 BELLEGARDE-EN-FOREZ (FR)**
• **LECOMTE, Serge**
**21000 DIJON (FR)**
• **ROBLOT, Magali**
**21160 PERRIGNY-LES-DIJON (FR)**

(74) Mandataire: **Cabinet Beau de Loménie**
**158, rue de l'Université**
**75340 Paris Cedex 07 (FR)**

(56) Documents cités:
**WO-A1-95/16416      WO-A1-2017/089731**

**Description**

Domaine Technique

La présente invention est relative à une bande de contention, qui est un tricot 3D obtenu selon la technologie maille jetée, sans latex ni adhésif, qui ne se détend pas ce qui permet de conserver son efficacité thérapeutique, et dont la surface en contact avec la peau a été modifiée pour optimiser son absence de glissement au cours du temps.

Technique antérieure

Le glissement d'une bande de contention est causé par 3 facteurs principaux. La qualité de pose, le glissement latéral spire sur spire et la capacité de la bande à résister au glissement sur la peau.

La demande de brevet WO 2017/089731 décrit les caractéristiques que doit posséder un tricot 3D, utilisé comme bande de contention, pour combiner efficacité thérapeutique et tenue dans le temps sans ajout de latex ou d'adhésif sur la bande.

Ainsi la capacité à éviter le glissement spire sur spire grâce à une contrainte de seuil de cisaillement supérieure ou égale à 2800 Pa et la sécurisation de la fixation finale de la bande à la fin de la pose permettent d'obtenir des produits qui remplissent ce cahier des charges.

Mais ces bandes demandent à être encore améliorées notamment sur deux points.

Le premier est lié à de la fabrication industrielle à grande échelle de tels tricots 3D car on obtient des bandes qui ne remplissent pas cette caractéristique de contrainte de seuil de cisaillement et qui vont donc se détendre et glisser lors de l'usage.

En effet on réalise du point de vue industriel non pas directement des bandes de largeur définies, par exemple 10 cm, mais des nappes de tricots 3D de plusieurs mètres de largeur et de longueur qui sont découpées ensuite en bobineaux de largeur définies et enfin des bandes finales en général de largeur de 8 ou 10 cm et d'environ 3 à 3,5 mètres de longueur. Lors de la fabrication de telles nappes et de ces opérations de découpe, on a sur de telles surfaces une variabilité du tricot 3D qui s'avère importante, et en particulier on obtient des bandes dont la valeur de la contrainte de seuil de cisaillement est inférieure ou proche de la valeur limite.

Le second est indépendant de la bande elle-même ; c'est le frottement d'un vêtement tel qu'un pantalon sur la bande lorsque cette dernière a été posée sur un patient. Ce phénomène apparait plus particulièrement quand, lors des mouvements l'écart entre la bande et le pantalon est faible.

Il existe selon les personnes une très grande variété de morphologie de jambes, notamment fines, larges, musculeuses, enrobées de graisse, osseuses, tronconiques avec une faible variation de diamètre entre le mollet et la cheville ou galbées avec des mollets proéminents.

C'est chez les patients qui présentent des jambes galbées avec des mollets proéminents que l'on constate principalement cette cause de glissement. Ce type de morphologie dans le cadre du traitement des ulcères de jambe ou de lymphoedème est rare car très souvent la présence d'oedème et l'absence d'activité physique à cause de la présence de la plaie entrainent que les patients présentent des jambes homogènes en diamètre et /ou peu musclées.

Ce type de morphologie de jambe peut être illustré par le rapport entre le point où le mollet à sa circonférence maximale et le point où la cheville a sa circonférence minimale. Quand ce rapport est supérieur à environ 1,6 le risque de glissement de la bande augmente.

Quand un mouvement est répété rapidement et très souvent, par exemple lors de la marche, on constate, à cause de ce frottement, au bout de quelques heures une très légère descente de la bande. Cependant, dans le cas de personne avec un mollet très galbé une fois la circonférence maximale du mollet dépassée, la bande étant toujours bloquée à cause de l'absence de glissement spire sur spire, elle se détend et finit par glisser. Ainsi plus le diamètre du mollet est important, plus le risque de frottement du pantalon sera élevé lors des mouvements.

Or un patient souhaite généralement cacher la présence de la bande et le port d'un pantalon dans un tel contexte est ainsi quasi systématique. Pour remédier à ce problème de frottement les patients recouvrent parfois la bande par une chaussette haute ou un bas pour éviter le contact avec le pantalon. Mais cette solution est souvent mal acceptée car l'ajout d'une couche supplémentaire augmente la chaleur ressentie par le patient.

Pour remédier à ces problèmes et obtenir un produit plus universel, qui permettrait de s'opposer au frottement du pantalon et d'éviter en fin de fabrication d'écarter des quantités de bandes importantes, avec une contrainte de seuil de cisaillement trop faible ou limite, le déposant a essayé d'optimiser ces tricots 3D.

Exposé de l'invention

Afin de répondre à ces problématiques, le déposant a dans un premier temps, essayé de modifier l'état de surface de la face venant en contact avec la peau pour augmenter la contrainte de seuil de cisaillement de ces tricots et en même temps le caractère agrippant de la face venant en contact avec la peau. L'objectif est de la rendre plus rugueuse, pour augmenter le frottement avec l'autre face, mais aussi plus agrippante avec la peau.

Diverses techniques sont connues pour augmenter la rugosité ou le caractère agrippant de la face d'un produit textile. Par exemple l'utilisation de fils réalisés dans des matériaux ayant un caractère agrippant relativement élevée tels des fils de silicone pour mieux adhérer à la peau. Une autre alternative est la création de boucles, en jouant sur la construction textile, qui dépassent de la surface et conduisent à une surface plus rugueuse au contact avec la peau et permettrait aussi ici d'augmenter le frottement avec l'autre face et donc la contrainte de seuil de cisaillement. Cependant, ces solutions complexifient la réalisation du tricot 3D car on modifie aussi en même temps les autres propriétés de la bande qui sont indispensables pour son bon fonctionnement et on augmente de manière significative son coût de production ce qui est rédhibitoire. La difficulté réside aussi dans le fait qu'au contraire, on vise à obtenir une face en contact avec la peau qui présente un toucher le plus doux possible car la peau des patients est souvent fragile ou altérée en particulier sur la zone sur laquelle la bande sera appliquée.

Contrairement à ce qui apparaissait logique et attendu le déposant a trouvé qu'il est avantageux de diminuer la rugosité de la face du tricot venant en contact avec la peau par traitement physique pour remplir ce cahier des charges complexe. Les bandes ainsi traitées, bien qu'elles possèdent des contraintes de seuil de cisaillement bien inférieures à 2800Pa et présentent une face venant en contact avec la peau plus douce que celles des tricots 3D décrits précédemment et donc moins agrippante, de façon surprenante, ne présentent pas de glissement spire sur spire et ne glissent pas au cours du temps.

Après une étude approfondie de cette situation inattendue le déposant a déterminé quelles caractéristiques la surface traitée devait présenter pour obtenir de telles propriétés.

On obtient ainsi une plus grande marge de manoeuvre lors de la fabrication industrielle des bandes, car on peut s'affranchir de la contrainte de seuil de cisaillement et les bandes sont aussi plus efficaces et mieux acceptées par le patient car la face venant en contact avec la peau est plus douce.

La présente invention est donc relative à une bande de contention telle que définie dans la revendication 1 qui se présente sous la forme d'un tricot obtenu par la technologie maille jetée, à base de fils synthétiques qui est constitué de 2 surfaces textiles dont la structure textile est identique ou différente reliées entre elles par un fil d'entretoise, chaque surface textile comportant des fils élastiques et le fil d'entretoise est un mono filament, ledit tricot présente un allongement longitudinal mesuré selon la norme EN 14704 -1 compris entre 30 et 160%, caractérisée en ce que la surface textile venant en contact avec la peau présente des fils multi filaments synthétiques, dont les filaments présentent un titre compris entre 1,2 et 5 dtex et le nombre de filaments de chaque fil multi filaments est compris entre 15 et 150, et la surface textile venant en contact avec la peau présente un coefficient de frottement dynamique mesuré selon la norme EN ISO 8295 supérieur ou égal à 0,25 et inférieur ou égal à 1,2, ou par exemple supérieur ou égal à 0,25 et inférieur ou égal à 0,5. La surface textile venant en contact avec la peau est typiquement une surface traitée physiquement, par exemple une surface émerisée.

Des tests décrits ci - après ont démontré qu'une bande possédant de telles caractéristiques permet de s'assurer du non glissement spire sur spire mais aussi de favoriser son caractère agrippant et de pouvoir s'opposer au glissement induit par le frottement du pantalon.

Selon la présente invention le tricot 3D pourra être à usage unique ou réutilisable et donc par conséquent lavable.

Afin de favoriser une pose précise par le personnel soignant, la bande de contention peut être munie d'un moyen d'étalonnage. Ce moyen d'étalonnage peut être visuel, comme par exemple un ensemble de pictogrammes, régulièrement espacés, imprimés sur la bande ou réalisés au moyen d'un système d'étalonnage. Des informations sur les allongements à la pose recommandés peuvent être fournies avec le moyen d'étalonnage. L'étalonnage peut aussi être réalisé par le personnel soignant sous la forme d'un pochoir. Ce type de pochoir ou les explications nécessaires pour le fabriquer peuvent être incorporés dans le conditionnement de la bande. Un kit comprenant plusieurs bandes de constitutions différentes, de largeurs différentes, de longueurs différentes et/ou dotées d'étalonnages différents pour appliquer des pressions spécifiques peut également être utilisé.

Le kit peut en outre comprendre un ou plusieurs pansements destinés à être posés sur la plaie avant la pose de la bande.

Pour favoriser la facilité de manipulation lors de la pose on choisira un tricot qui présente un allongement longitudinal tel que défini dans la norme EN 14704-1 qui soit compris entre 40 et 160%, ou plus précisément entre 65 et 120 %, ou encore plus précisément entre 70 et 95%.

Le tricot présente par exemple une épaisseur comprise entre 1 et 2 mm, ou plus précisément entre 1 et 1,5mm.

Le tricot présente par exemple un grammage compris de 160 à 370 g/m$^2$, ou plus précisément de 180 à 300 g/m$^2$, ou encore plus précisément de 200 à 250 g/m$^2$.

Le tricot présente par exemple un écart entre les 2 faces textiles compris entre 0,4 et 1,5mm, ou plus précisément entre 0,5 et 1,1mm.

Ces propriétés de faible grammage et d'épaisseur assurent une utilisation facilitée avec les chaussures de la bande de contention. La bande de contention pourra ainsi être aussi plus facilement utilisée avec une ouate si nécessaire. Elles permettent aussi de diminuer les risques de frottement avec le pantalon car la bande est très fine.

Les deux surfaces textiles du tricot peuvent avoir des structures textiles identiques ou différentes. Ces structures textiles peuvent être pleines ou ajourées.

Les structures textiles ajourées appelées tricot à jours et désignées dans la présente demande sous le terme de filet sont bien connues par l'homme du métier.

Un tricot à jours est un tricot qui présente des trous réguliers ou irréguliers dans sa structure textile. Ces trous sont obtenus lorsque, dans la structure textile, une ou plusieurs mailles d'une colonne ne sont pas reliées aux mailles de la colonne voisine lors du tricotage, typiquement en jouant sur le schéma de maille et / ou sur l'enfilage.

Selon un aspect de la présente invention le tricot présente deux surfaces textiles dont la structure textile est différente et en particulier une surface textile qui présente une structure textile ajourée appelée face filet et une surface textile qui présente une structure textile face pleine. La présence d'une face filet permet de favoriser la respirabilité de la bande. Une telle face filet est typiquement disposée au contact de la peau d'un utilisateur.

Selon un mode de réalisation particulier, ledit tricot présente une face qui a une structure textile de type charmeuse, drap, demi-simple à mailles ouvertes ou fermées, atlas sous un ou plusieurs rangs, ou chainette à mailles ouvertes, fermées, ou à alternance de mailles fermées et ouvertes. Cette face est opposée à la face adaptée pour être mise en contact avec la peau, qui présente une structure textile qui est un filet avec le même type ou un type différent de structure textile mais ajourée.

Afin de faciliter le passage du talon et éviter une striction de la bande lors de la pose on peut utiliser des tricots 3D qui présentent un allongement transversal supérieur à 110 % tel que mesuré selon la méthode A §9.2 de la norme EN 14704-1, ou par exemple compris entre 110 % et 200 %, ou encore entre 120 % et 180 %.

Les tricots selon l'invention sont par exemple réalisés à l'aide de fils couramment employés dans la réalisation des produits textiles et en particulier des tricots. Ces fils sont par exemple synthétiques. Ces fils se partagent en 2 grandes catégories : les fils élastiques et les fils thermoplastiques.

Parmi les fils élastiques on peut par exemple citer les fils à base de fibres polyuréthanne comme les fils d'Elasthanne commercialisés sous la dénomination LYCRA, les fils à base d'élastodiène ou les fils à base de polymères triblocs (styrène - éthylène - butylène - styrène).

Parmi les fils thermoplastiques on peut citer les fils constitués à base de matériaux synthétiques qui ne sont pas des élastomères comme par exemple le polyester, le polyamide, le polypropylène, le polybutylène terephtalate (PBT).

Tous ces fils thermoplastiques peuvent être guipés ou non, texturés ou non.

Les deux surfaces textiles du tricot 3D sont par exemple réalisées à partir de fils élastiques et de fils thermoplastiques. Ces fils peuvent être mono filaments ou multi filaments. Ces surfaces textiles pourront être réalisées à partir de fils identiques ou différents. Les deux surfaces comprendront typiquement des fils élastiques similaires.

Les fils élastiques présents sur ces surfaces textiles présentent par exemple des titres de l'ordre de 40 à 80 dtex et les fils thermoplastiques des titres de 40 à 90 dtex.

La face venant en contact avec la peau contient obligatoirement des fils thermoplastiques qui sont des multi filaments synthétiques. Afin d'obtenir après traitement mécanique le bon coefficient de friction dynamique ces multi filaments présentent des filaments dont le titre est compris entre 1,2 et 5 dtex et le nombre de filaments est compris entre 15 et 150.

Ces fils sont par exemple en polyamide, polyester ou polypropylène. Ils peuvent présenter un titre compris entre 25 et 200 dtex. Dans le cadre de la présente invention on utilise typiquement des fils multi filaments en polyamide qui présentent un titre compris entre 40 et 90 dtex.

Cette face ne contient pas de fils de nature non synthétiques, comme par exemple le coton ou la viscose, car les fils naturels ne résistent pas au traitement physique.

On utilise sur la face venant en contact avec la peau une quantité de fils thermoplastiques multi filaments de l'ordre de 15 à 40% en pourcentage massique par rapport au poids total du tricot 3D et par exemple de l'ordre de 20 à 25%. La proportion de fils complémentaires sont des fils élastiques.

Si l'on souhaite favoriser le transfert d'humidité du tricot vers l'extérieur on pourra utiliser des fils de nature non synthétique sur la face ne venant pas en contact avec la peau.

On utilise par exemple comme fils élastiques des fils d'élasthanne et comme fils thermoplastiques des fils de polyamide ou de polyester.

Les fils d'entretoises sont typiquement des fils thermoplastiques mono filament, comme par exemple des fils en polyester, en polypropylène ou en polyamide.

Selon un exemple, les fils d'entretoises sont des fils en polyester ou en polyamide qui présentent par exemple un titre compris entre 20 et 80 dtex , ou encore entre 40 et 70 dtex, ou encore plus précisément un mono filament en polyester qui présente un titre entre 44 et 55 dtex.

Pour la réalisation du tricot 3D, on peut par exemple utiliser une seule barre pour tricoter le fil d'entretoise qui lie les 2 surfaces textiles.

L'invention concerne également un kit comprenant une ou plusieurs bandes de contention telles que définies précédemment, et un ou plusieurs pansements adaptés pour être disposés sur une plaie préalablement à l'une des bandes de contention.

Le traitement physique de la surface venant en contact avec la peau sera réalisé dans une étape ultérieure après le tricotage. L'objectif est de modifier l'état de la surface en altérant les multi filaments. On peut citer à titre d'exemple comme traitement physique par exemple le grattage, le brossage ou l'émerisage. Dans le cadre de l'invention on utilise typiquement l'émerisage. Cette opération, bien connue dans le domaine de l'habillement, consiste à figer par la chaleur la structure textile avec une température de l'ordre de 190°c puis à passer cette dernière, une ou plusieurs fois, sous un rouleau de toile émeri. Bien évidemment toute opération technique qui permettrait d'obtenir une face venant en contact avec la peau présentant les bonnes caractéristiques pourra être employée.

Ces procédés de traitement de la surface d'un matériau textile sont couramment utilisés.

Mais à la connaissance du déposant personne n'a jamais étudié et évalué l'impact d'un tel traitement pour favoriser l'absence de glissement spire sur spire ou l'effet que l'on qualifie d'accroche à la peau.

Le document EP 1 052 319 décrit un tricot 3D utilisable dans des dispositifs orthopédiques qui entoure, par exemple une articulation comme illustré sur la figure 7 du document EP 1 052 319, mais jamais une bande de contention pour laquelle on superpose 2 couches de tricot 3D l'une sur l'autre pour bander la jambe. Le problème du glissement spire sur spire ne se pose donc pas dans ce document.

Les tricots 3D développés dans le cadre du présent exposé sont très différents des tricots 3D existant et notamment de ceux décrits dans le document EP 1 052 319 mentionné précédemment, car la surface des tricots 3D connus accroche avec les crochets d'un dispositif type Velcro ®, par opposition à la surface des tricots 3D développés dans le cadre du présent exposé qui, après traitement, n'accroche plus avec lesdits crochets.

Dans le cadre de la présente invention, on traite, à cause de cette absence d'accroche des crochets, uniquement la face venant en contact avec la peau car en fin d'enroulement la bande est typiquement bloquée sur ces 2 dernières spires à l'aide d'un tel dispositif avec crochets. De plus la face opposée à celle venant en contact avec la peau est celle sur laquelle on imprime typiquement l'étalonnage pour faciliter la pose de la bande et il peut donc être avantageux de ne pas traiter cette dernière pour ne pas altérer l'impression.

L'état de surfaces des tricots 3D traités est donc totalement différent de celui recherché dans EP 1 052 319.

L'observation microscopique des surfaces traitées des tricots 3D selon l'invention montre que tout ou partie des fibres des multi filaments sont désordonnées, désunies, râpées, présentent des aspérités, moins lisses et coupées par rapport au tricot 3D avant traitement ce qui explique l'absence d'accroche des crochets.

Cet état de surface de la face traitée explique sans doute aussi pourquoi on diminue de façon significative la valeur de la contrainte de seuil de cisaillement par rapport aux exemples de la demande WO 2017/089731. Mais de façon non expliquée ceci conduit néanmoins à un produit performant en termes de blocage du glissement spire sur spire.

On constate aussi comme l'illustrent les tests décrits ultérieurement que l'on optimise la résistance au glissement sur la peau.

Mais l'observation microscopique de la surface de la face traitée ne permet de déterminer un nombre ou une forme de filaments déstructurés indispensables pour quantifier cette absence de glissement spire sur spire et l'accroche à la peau de la bande.

Dans le cadre du présent exposé, on a donc cherché à caractériser l'état de surface général de telles bandes.

Dans cette optique le déposant a étudié le coefficient de frottement dynamique de la face venant en contact avec la peau qui a été traitée.

Le coefficient de frottement dynamique caractérise l'aptitude d'une surface à glisser sur elle-même ou une autre surface. La mesure de ce paramètre, couramment utilisée dans le domaine des films est décrite dans la norme EN ISO 8295.

La demande WO 2014/033418 suggère l'utilisation de cette norme pour mesurer la liaison entre 2 faces d'une bande de contention. Mais dans ce cas on constate une accroche physique et non pas un frottement entre les 2 faces car les boucles présentes en surface s'entremêlent les unes dans les autres. Ceci conduit à des valeurs de coefficient de friction dynamique supérieure à 3. Mais dans le cadre de la présente invention il n'y a pas d'accroche physique entre les 2 faces d'un même matériau comme dans WO 2014/033418. Le terme accroche illustre ici uniquement un frottement entre la peau et la face du tricot venant en contact avec la peau.

La principale difficulté réside ici dans le fait que l'on souhaite mesurer un tel coefficient entre la peau et la face d'un tricot 3D qui peut présenter une variabilité importante. Or, la peau est particulièrement difficile à caractériser, et il est difficile de définir un matériau équivalent.

Il est donc indispensable de trouver un matériau de référence qui puisse simuler la peau et être discriminant entre les tricots 3D au niveau de la caractérisation de leur état de surface.

A la connaissance du déposant il n'existe aucune méthode pour mesurer l'accroche à la peau d'une bande in vivo ou in vitro. La mise au point d'un modèle de peau est très complexe et il n'existe pas aujourd'hui un modèle représentatif de la peau pour évaluer cette propriété. De plus il existe une multitude de peaux ayant des propriétés distinctes, notamment couleur, présence de poils, grasses, sèches, altérées.

Enfin, pour être le plus représentatif, il faut se placer dans les conditions d'utilisation de la bande qui est posée en extension et conduit à un équilibre de force entre la pression appliquée et la force de frottement entre la bande et la peau.

Pour se rapprocher de la réalité et pour trouver un matériau de référence le déposant a mis au point un test pour évaluer le frottement d'une bande sur la peau dans des conditions qui illustrent les conditions d'utilisation de la bande posée en extension.

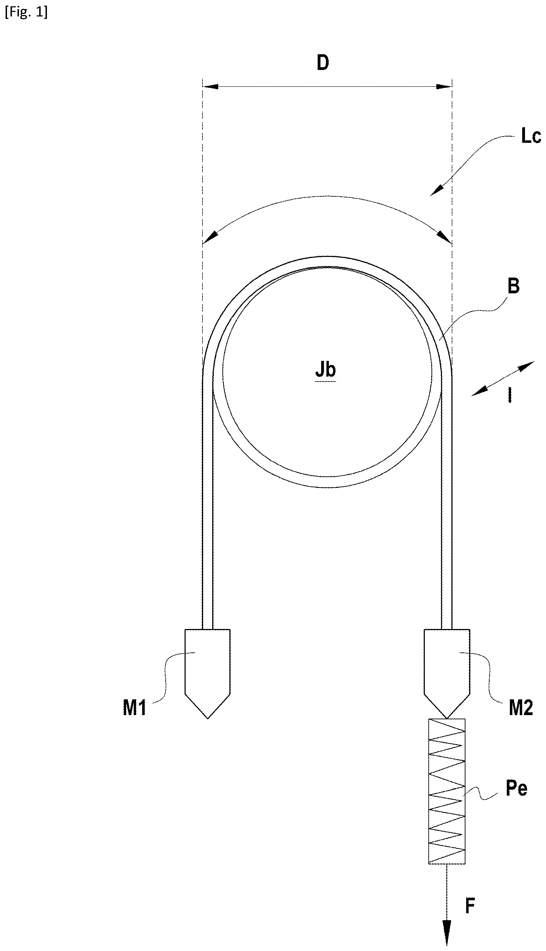

Ce test est décrit en référence à la figure 1, sur laquelle on représente schématiquement la jambe Jb d'un patient par un rond autour duquel on vient positionner une bande B.

L'évaluation de ce frottement illustre la résistance que la peau et la bande en contact l'une avec l'autre opposent au glissement. On va déterminer la force de frottement minimum qu'il faut vaincre pour enclencher un début de glissement. Le coefficient de frottement correspond au rapport des 2 forces qui s'appliquent ici : la force nécessaire pour vaincre le mouvement que l'on mesure et la force de contact imposée par la bande en extension qui correspond à la pression appliquée par les 2 masses à l'équilibre sur la surface de la bande en contact avec la peau Sc (Sc = Le * I, où Le est la longueur de la bande en contact avec la peau et I est la largeur de la bande).

La description du test est la suivante.

Le testeur est assis et pose son talon sur un support, par exemple une chaise, de manière à avoir la jambe droite selon un axe horizontal.

On découpe un échantillon de bande longitudinalement sur une largeur de 5 cm et sur 50 cm de longueur.

On applique l'échantillon de bande sur la face opposée au mollet au niveau de la circonférence du mollet la plus importante de manière à avoir des longueurs de bandes équivalentes de chaque côté de la jambe comme illustré sur la figure 1.

A chaque extrémité sur la largeur de bande on fixe comme illustré sur la figure 1 deux masses M1 et M2 de 500 g pour exercer une force de pression du même ordre de grandeur (de l'ordre ici de 35 mm de mercure) que la pression exercée par la bande sur la jambe quand cette bande est en extension. La face du tricot en contact avec la jambe est la face qui sera en contact peau en usage normal du produit. Les forces de frottements bande-peau maintiennent immobiles les 2 masses. On dispose un peson Pe à l'une des extrémités de la bande sous la masse.

L'objectif du test consiste à tirer côté peson manuellement légèrement et progressivement sur le peson jusqu'à ce que l'on induise le mouvement de glissement de la bande et d'enregistrer la force F qui permet ainsi de mettre en mouvement l'ensemble c'est-à-dire la bande et les masses. La force est mesurée directement avec un peson conventionnel étalonné et équipé d'un index pour mesurer cette force.

Grâce à la connaissance des masses M1 et M2 (qui sont identiques et égales à M) et de la force F, on peut par calcul estimer avec la formule Math.1 ci - dessous (en supposant que la jambe est cylindrique et que le diamètre D de la jambe et la longueur de contact Le sont identiques) le coefficient de friction $\mu$ de la bande sur la peau.

[Math. 1]

$$\mu = \frac{F}{2 \times M \times g + F} \times \frac{2}{\pi}$$

Où F est exprimé en Newtons, M en kg et g = 9,81 m/s/s. La jambe du patient est ici considérée comme un cylindre parfait ; cette approximation n'ayant que peu d'incidence sur le résultat.

La valeur moyenne, sur la base de ce test, du coefficient de frottement des tricots 3D selon l'invention sur la peau est de 0,28.

Mais pour sélectionner ou caractériser des bandes en fin de production un test in vivo est inadapté. Pour trouver un matériau de référence utilisable dans le test vitro décrit dans la norme EN ISO 8295 dans une seconde étape le déposant a reproduit ce test mais en déposant au préalable autour de la jambe à l'endroit où l'on va poser la bande un échantillon, qui va entourer la jambe sur la surface qui va être recouverte par la bande lors du test, de matériau de référence, fixé à l'aide d'un adhésif double face, sur lequel on va poser l'échantillon de bande. Le test conduit alors à la mesure du coefficient de friction de la bande sur le matériau.

Grâce à cette approche il a cherché à trouver un matériau, qui serait utilisable dans un test in vitro, pour caractériser l'état de surface des bandes et pouvoir si nécessaire sélectionner les bandes qui présentent le bon état de surface.

Le déposant a testé alors divers matériaux pour essayer d'en trouver un qui présente le même résultat de coefficient de frottement. Il n'en a pas trouvé, mais il a déterminé un matériau plus discriminant sur lequel le coefficient de frottement donne une valeur de 0,19.

Ce matériau est un non tissé à base de polyéthylène haute densité qui présente un grammage de 82g/m$^2$ et une face lisse, sur laquelle on vient poser lors du test la face du tricot 3D qui vient en contact avec la peau, commercialisé par la société DuPont de Nemours sous la dénomination commerciale « Tyvek ® ». Ce matériau est utilisé en matière d'étanchéité sous les toitures et les sous couches pour murs et cloisons extérieures. Sa référence est TYVEK ® TOITURE 45.

Il a donc choisi ce dernier comme matériau de référence pour mettre en oeuvre la norme précitée.

Grâce à l'utilisation comme matériau de référence pour ce test on peut ainsi caractériser et quantifier l'état de surface des tricots 3D et obtenir une valeur discriminante corrélée avec l'absence de glissement spire sur spire et le non glissement de la bande. On a ainsi pu déterminer que la surface doit présenter un coefficient de frottement dynamique supérieur ou égal 0,25 et inférieur ou égal à 1,2.

En effet on considère que pour un coefficient de frottement dynamique supérieur à 1,2, on ne mesure pas avec le test selon la norme EN ISO 8295 un frottement entre 2 matériaux mais une accroche physique entre ces 2 matériaux comme dans le document WO 2014/033418.

Dans le cadre du présent exposé on utilise typiquement des tricots 3D qui présentent un coefficient de friction dynamique compris entre 0,25 et 0,8 et par exemple entre 0,3 et 0,5.

On a aussi déterminé que pour obtenir à la fois un aspect doux mais aussi des propriétés suffisantes d'accroche à la peau qui conduisent à une surface qui présente cette caractéristique il est nécessaire que les multi filaments en surface de la face venant en contact avec la peau présentent eux aussi des caractéristiques spécifiques.

Ces multi filaments doivent être synthétiques car les fils naturels ne résistent pas aux traitements physiques. Les fils multi filaments qui conviennent sont ceux dont les filaments présentent un titre compris entre 1,2 et 5 dtex et le nombre de filaments est compris entre 15 et 150.

Il semble en effet que la déstructuration des fils synthétiques à base de multi filaments dans de telles gammes de valeurs permet d'obtenir en surface de la bande des filaments suffisamment fins pour ne pas agresser la peau mais augmenter le frottement, quand la face en contact avec la peau est traitée, et suffisamment longs pour favoriser le frottement entre les spires.

La présente invention est donc relative à des bandes, dont l'allongement longitudinal est compris entre 30% et 160%, qui sont des tricot 3D, obtenu selon la technologie maille jetée, sans latex ni adhésif dont le fil d'entretoise est un mono filament, qui présentent sur leur surface venant en contact avec la peau des fils multi filaments synthétiques, dont les filaments présentent un titre compris entre 1,2 et 5 dtex et le nombre de filaments de chaque fil multi filaments est compris entre 15 et 150, lesdits fils multi filaments étant par exemple déstructurés par opération mécanique de manière à ce que la face (ou surface textile) venant en contact avec la peau présente un coefficient de frottement dynamique supérieur ou égal à 0,25 et inférieur ou égal à 1,2, qui ne glissent pas au moins pendant 48 heures et mieux au moins pendant 3 jours ou plus.

En effet dans le cadre du traitement des ulcères de jambes qui présentent des plaies très exsudatives ces durées minima de 48 et 72 heures correspondent aux durées habituelles de changement des pansements qui sont disposés sous les bandes de contention.

Brève description des dessins

L'invention et ses avantages seront mieux compris à la lecture de la description détaillée faite ci-après de différents modes de réalisation de l'invention donnés à titre d'exemples non limitatifs.

[Fig. 1] La figure 1 déjà décrite précédemment représente schématiquement un dispositif de test pour évaluer le frottement d'une bande sur la peau.

[Fig. 2] La figure 2 représente un exemple de structure de maille pour la réalisation d'un tricot selon le présent exposé.

Description des modes de réalisation

On décrit ci-après un exemple de mise en oeuvre de l'invention.

**Etape 1.**

On a fabriqué une nappe de tricot d'environ 40 cm de largeur selon l'invention sur un métier Raschel maille jetée double fonture jauge 22.

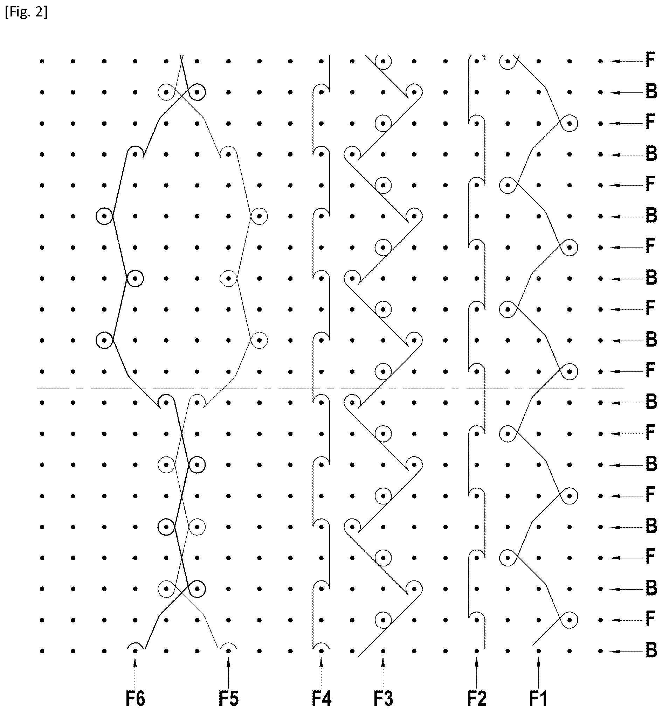

Pour réaliser cette nappe on a utilisé 6 barres selon le schéma de maille représenté sur la figure 2 avec les fils et les conditions suivantes :

**Nature des fils :**

- F1 : fil de polyamide commercialisé par la société RADICI sous la référence 78/18/1 dtex S Beige.
- F2 : fil d élasthanne de 44 dtex commercialisé par la société ASAHI KASEI GROUP.
- F3 : fil qui est un monofilament de polyester de 55 dtex commercialisé par la société FILVA.
- F4 : fil d'élasthanne de 44 dtex commercialisé par la société ASAHI KASEI GROUP.
- F5 : fil de polyamide 66 commercialisé par la société DEFIBER SA sous la référence PA 66 1/ 44/34.
- F6 : fil de polyamide 66 commercialisé par la société DEFIBER SA sous la référence PA 66 1/ 44/34.

**Réglages du métier à tricoter**

- F1 ; alimentation 2650 mm de fils consommés pour la réalisation de 480 mailles, enfilage plein,
- F2 : alimentation 1170 mm de fils consommés pour la réalisation de 480 mailles, enfilage 1 plein / 1 vide,
- F3 : alimentation 3700 mm de fils consommés pour la réalisation de 480 mailles, enfilage plein,
- F4 : alimentation 1270 mm de fils consommés pour la réalisation de 480 mailles, enfilage 1 plein / 1 vide,
- F5: alimentation 2150 mm de fils consommés pour la réalisation de 480 mailles, enfilage 3 pleins / 1vide,
- F6 : alimentation 2150 mm de fils consommés pour la réalisation de 480 mailes, enfilage 3 pleins / 1 vide.

**Schéma de maille.**

On représente sur la figure 2 le graphique d'un exemple de structure de maille pour la réalisation d'un tricot selon un mode de réalisation particulier de l'invention.

Sur cette figure, on représente la fronture avant par la référence F, et la fronture arrière par la référence B. Les schémas de maille des fils F1 à F6 sont ensuite illustrés.

On comprend bien que cet exemple est purement illustratif, et qu'il ne doit pas être interprété de manière limitative quant à la portée de l'invention.

La nappe ainsi réalisée subit une étape de découpe pour réaliser des bandes de 10 cm de largeur.

### Traitement physique.

Une nappe de 40cm de largeur obtenu comme lors de l'étape 1 est alors traitée physiquement sur sa face venant en contact avec la peau, par exemple par émerisage. La surface venant au contact avec la peau est ainsi typiquement une surface émerisée.

Durant cette étape le tricot subit plusieurs étapes de traitement. Il est d'abord traité à 190 °C à la vitesse de 10m/mn puis passe en contact successivement avec 2 rouleaux de grains Emeri le premier avec des grains de 280 et le second de 400 suivi d'une dernière étape de chauffage à 120°C à la même vitesse que la première étape.

La nappe ainsi traitée subit une étape de découpe pour réaliser des bandes de 10 cm de largeur.

Les techniques suivantes ont été utilisées pour évaluer les paramètres des bandes obtenues.

### Mesure du grammage.

La mesure du grammage est réalisée selon la norme NF EN 12127. On pèse 5 éprouvettes d'une surface de 1000 cm$^2$ (mesure = +/- 1 %) avec une balance dont la précision est au plus de 1 mg.

La pesée est réalisée à une température de 21 °C +/-2 °C et 60 % +/- 15 % H R.

La mesure finale est une moyenne des 5 éprouvettes.

### Mesure de l'épaisseur.

La mesure de l'épaisseur s'effectue selon la norme NF EN ISO 9073-2. On utilise un micromètre TESTWELL DM 100. La pression d'application est fixée à 0,5kPa et la surface du disque en acier est de 2500mm$^2$.

La mesure finale est une moyenne de 3 éprouvettes de bande.

### Mesure de l'écart entre les faces.

Cette mesure est réalisée de la façon suivant.

A l'aide d'un microscope numérique KEYENCE (optiques x 100 ou x 200) on détermine l'espace entre les deux plans des 2 surfaces textiles.

Le plan moyen des 2 surfaces est matérialisé par un trait horizontal estimé par l'opérateur et la distance entre les deux traits est déterminée de manière automatique par le logiciel .La mesure est reproduite plusieurs fois et on réalise une moyenne des mesures obtenues.

### Mesure de la contrainte seuil de cisaillement.

Les mesures sont réalisées à l'aide d'un rhéomètre DHR 2 commercialisé par la société TA Instruments.

Elles sont effectuées à une température de 35°C (de manière à être proche de la température des bandes au contact de la peau) ladite température étant régulée par un plan Peltier qui équipe le rhéomètre.

On découpe 2 disques de 25 mm de diamètre du tricot 3D analysé.

Ces 2 disques sont collés respectivement à l'aide d'un adhésif double face fin et rigide commercialisé par la société PLASTO sous la référence P753 sur la face métallique du plateau mobile et du plateau du plan Peltier du rhéomètre. On met en contact les 2 disques de tricot 3D, face structure charmeuse (également appelée structure drap) sur face structure filet, en appliquant une pression de 5,3 kPa (soit l'équivalent de 40mm de mercure). Le programme de pilotage du rhéomètre génère une rampe de contrainte (couple de torsion) qui varie de 100 à 10 000 Pa en 600 secondes. L'appareil enregistre le premier micro-déplacement qu'il détecte qui correspond à la contrainte seuil de cisaillement exprimée en Pa.

On considère que l'incertitude instrumentale sur cette mesure est plus ou moins 6%.

La mesure finale est la moyenne des valeurs obtenues pour 5 échantillons de la même bande de tricot 3D.

### Mesure du coefficient de frottement dynamique.

La mesure du coefficient dynamique est basée sur le principe décrit dans la norme EN ISO 8295. Elle utilise comme matériau de référence le produit commercialisé par la société DuPont de Nemours sous la dénomination Tyvek ® dont la référence est TOITURE 45. Ce matériau est un non tissé à base de polyéthylène haute densité qui présente un grammage de 82g/m$^2$ et une face lisse, sur laquelle on vient poser lors du test la face du tricot 3D venant en contact avec la peau. Elle consiste à mesurer la force pour déplacer sur un grand plateau à l'aide d'un dynamomètre électronique un patin carré en acier de 200 g de 63,5 mm de longueur et 63,5 mm de largueur, lequel est recouvert de la bande de contention côté face venant en contact avec la peau vers l'extérieur, qui glisse sur le grand plateau qui est lui aussi recouvert sur l'ensemble de sa surface par du Tyvek ® la face lisse vers l'extérieur de la même façon. La surface de la bande venant en contact avec la peau est donc en contact avec la face lisse du Tyvek ® et le patin est tiré à la vitesse de 100 mm/min par le dynamomètre. La bande et le Tyvek ® sont collés sur le patin en acier et sur le plateau à l'aide d'un adhésif double face. Le patin est relié par un câble au capteur de force du dynamomètre. La mesure du coefficient de frottement dynamique est le rapport entre la force de traction, exprimée en Newtons, mesurée par le dynamomètre et le poids du patin, exprimé en Newtons, soit 1,96 N tel que défini dans la norme. On réalise la mesure sur trois échantillons découpés sur la même bande et on prend la valeur moyenne de ces trois mesures.

**Mesure de l'allongement longitudinal et transversal.**

Elle est basée sur la mesure de l'allongement longitudinal de la bande telle que définie dans la méthode A §9.2 de la norme EN 14704-1 quand la bande est soumise à une force de traction maximale de 5 N/cm.

Les conditions de réalisation de la mesure sont les suivantes.

Une éprouvette du matériau à tester de 100 mm de largeur et de 150 à 200 mm de longueur est découpée et positionnée sans précontrainte dans les mors d'un dynamomètre électronique (par exemple un dynamomètre commercialisé par la société MTS Systems corporation) de sorte à avoir une largeur de 100 mm et une longueur utile de référence de 100mm. Le dynamomètre étire l'éprouvette à une vitesse de 100 mm/min jusqu'à une force maximale de 5 N/cm puis la traverse revient à sa position initiale à la même vitesse de retour de 100 mm/min. Ce cycle est effectué 5 fois et l'allongement obtenu au cinquième cycle, exprimé en pourcentage, est directement calculé par l'appareillage. L'opération est répétée sur 5 éprouvettes, puis on calcule la valeur moyenne qui définit l'allongement longitudinal de la bande.

L'allongement transversal de la bande est évalué selon le même protocole mais en adaptant les dimensions de l'éprouvette à la largeur de la bande qui est de 10 cm. On utilise donc une éprouvette de 60mm de longueur et de 50mm de largeur.

Les paramètres des bandes obtenues sont les suivants.

Bande non traitée :

- Grammage : 238 g/m$^2$
- Epaisseur : 1,6 mm
- Contrainte seuil de cisaillement : 2947 Pa
- Ecart entre les faces : 0,88 mm
- Allongement longitudinal selon la norme EN 14704 - 1 : 80 %
- Allongement transversal selon la norme EN 14704 - 1 : 144 %
- Coefficient de frottement dynamique selon la norme EN ISO 8295 : 0,22.

Bande traitée :

- Grammage : 202 g/m$^2$
- Epaisseur : 1,2 mm
- Contrainte seuil de cisaillement : 2357 Pa
- Ecart entre les faces : 0,74 mm
- Allongement longitudinal selon la norme EN 14704 - 1 : 84 %
- Allongement transversal selon la norme EN 14704 - 1 : 150 %

- Coefficient de frottement dynamique selon la norme EN ISO 8295 : 0,34.

On a vérifié à l'aide du test suivant pour les 2 bandes ci - dessus la tenue au cours du temps de la bande.

On a sélectionné parmi les testeurs habituels celui chez qui on a le plus grand nombre de glissement de bandes lors des tests. Il présente des mollets très musclés et galbés ce qui se traduit par un rapport entre le point où le mollet a sa circonférence maximale et le point où la cheville a sa circonférence minimale de l'ordre de 1,7.

Le mode opératoire de ce test in vivo est le suivant.

On enroule la ou les bandes de contention autour de la jambe selon les préconisations décrites dans la notice du système bicouches URGO K2 ®, qui comprend deux bandes distinctes ; une bande dite KTECH et une bande dite KPRESS.

Pour rappel, cette notice préconise la méthode d'application suivante :

1) tenir le pied à 90°, orteils vers le haut ; appliquer KTECH à la base des orteils en faisant deux tours d'ancrage, en s'assurant que la face ouatée soit en contact direct avec la peau et que l'indicateur de pression soit situé sur le côté supérieur de la bande ; continuer en faisant une figure en « 8 » autour de la cheville, sans appliquer une tension excessive sur le pied et en recouvrant bien le talon ;
2) remonter jusqu'au genou en effectuant des spirales et en étirant la bande de manière appropriée : l'indicateur de pression imprimé sur les bandes doit former un cercle ; pour obtenir un recouvrement correct, l'indicateur de pression doit être tout juste recouvert (recouvrement de 50%) ; finir 2cm sous le genou et couper l'excédent de bande ;
3) appliquer KPRESS sur KTECH selon la même technique en commençant un doigt au-dessus de KTECH et en finissant un doigt sous KTECH afin que seule KTECH soit en contact direct avec la peau ; une fois appliquée, appuyer doucement sur le bandage avec les mains pour assurer une bonne tenue du système.

On comprend bien que cette dernière étape 3) n'est pas nécessaire si on n'utilise pas une seconde bande de contention auto adhérente. Dans ce cas, la dernière spire est fixée sur elle-même à l'aide de la partie à crochets d'un Velcro ®. Toutefois, on appuie à nouveau doucement sur le bandage avec les mains pour assurer une bonne tenue du système de contention.

Pour tester les bandes de contention des exemples précédents on a déterminé leur allongement à la pose comme indiqué dans la demande de brevet WO2017/089731 page 28 ligne 21 à page 29 ligne 13. Ainsi, pour tester les bandes de contention selon l'invention on a déterminé l'allongement à la pose de la bande en fonction de la pression de travail recherchée, de l'ordre de 60 à 70 mm de mercure, par exemple à l'aide de la courbe traction rupture telle que définie dans la norme EN ISO 13934-1. D'après la loi de Laplace, l'allongement à effectuer correspond à la pression recherchée. Le principe de cette norme est le suivant. On découpe une bande rectangulaire de largeur suffisante en l'effilochant si besoin pour obtenir un échantillon de largeur finale de 50mm. On place cet échantillon dans les mors d'un dynamomètre distants de 200mm. On procède à l'essai de traction jusqu'à la rupture de l'échantillon à la vitesse de 100mm/mn. On répète ainsi le test pour 5 échantillons. Les conditions de conditionnement, d'hygrométrie et de température sont définies dans la norme EN ISO 13934 - 1.

Pour poser la bande de façon appropriée, on a étalonné les bandes à l'aide d'un pochoir comme décrit dans la demande de brevet WO 2007/113340 page 13, ligne 18 à page 14, ligne 6.

On a déterminé un allongement à la pose de 45% et on a réalisé l'étalonnage correspondant.

On trace à l'aide d'un feutre fin et non effaçable un trait vertical sur au moins trois spires, sur l'axe de la crête tibiale, à partir de la dernière spire enroulée. Cette marque sert de référence pour évaluer à l'aide d'un réglet gradué au mm, le décalage horizontal du trait au terme de la durée du test. Lors des mouvements, ce trait perd de son caractère rectiligne et se présente en échelons d'autant plus décalés que les glissements spires sur spires sont importants. Si le glissement spire sur spire est très faible ou inexistant, le trait vertical reste intègre ou varie très peu principalement sur la première spire qui se situe sous la dernière spire enroulée.

Ce décalage du trait vertical est représentatif du relâchement de la bande et illustre son glissement potentiel au cours du temps. Le test dure 6 heures. On mesure au bout des 6 heures le décalage du trait vertical sur les 3 premières spires.

On trace aussi à l'aide du feutre fin et non effaçable un trait horizontal sur la peau au - dessus de la dernière spire enroulée. Cette marque sert de référence pour évaluer à l'aide d'un réglet gradué au mm, le décalage vertical entre ce trait et la position sur la jambe de la dernière spire au terme de la durée du test. On considère que si l'écart entre le trait horizontal et la dernière spire est, au bout de 6 heures, supérieur à 15 mm la bande glissera complètement avant 24 heures.

Durant les 6 heures le testeur a mené une activité habituelle sans effort spécifique.

On a réalisé un premier test dans lequel le testeur porte sur chaque jambe une bande non traitée.

La jambe gauche présente un rapport de différence de circonférence de 38/22 soit 1,73 et la jambe gauche de 38/22,5 soit 1,68.

Au bout de 6 heures on constate sur la jambe gauche une baisse du trait horizontal de 17mm et un décalage entre la première spire et la seconde de 6mm et entre la seconde et la troisième de 8mm. Sur la jambe droite une baisse du trait horizontal de 2mm et un décalage de 2mm entre la première spire et la seconde et aucun décalage entre la seconde et la troisième spire.

Dans le cas de la jambe droite on a le comportement classique d'une bande décrite dans WO2017/089731 qui présente une contrainte de cisaillement supérieure à 2800 Pa, ici 2947 Pa, et on ne constate pas de glissement spire sur spire ou le long de la jambe significatif.

A l'inverse dans le cas de la jambe gauche au bout de 6 heures la bande présente un décalage du trait horizontal de 17mm et des décalages spire sur spire qui augmente quand on descend le long de la jambe. Ceci illustre l'influence du frottement du pantalon dans le cas d'un mollet galbé. On a sans doute un frottement du pantalon sur la bande qui l'a fait descendre légèrement au cours du temps. Une fois la circonférence maximale du mollet dépassée la bande commence à se détendre ce qui est illustré par le décalage spire sur spire constaté qui va entraîner alors un glissement plus important de la bande. Cette bande va glisser complétement rapidement.

On a réalisé un second test similaire avec le même testeur mais avec cette fois une bande traitée sur chaque jambe.

En revanche ces bandes étant supposées plus performantes en terme de glissement et d'accroche à la peau on a durcit les conditions de test. On a introduit une activité physique intense en ajoutant 1 heure 30 de marche continue sur un tapis après la pose et 30 minutes supplémentaires de marche au bout de 4 heures. Enfin le testeur a porté un pantalon type « jeans » classique en denim susceptible de favoriser le frottement, et le test a duré 7 heures.

Au bout de 7 heures on n'a constaté aucun décalage spire sur spire sur aucune des 2 jambes et des décalages verticaux minimes de 0,3 mm sur la jambe droite et 0,6 mm sur la jambe gauche.

Ces bandes traitées remplissent donc le cahier des charges visé même dans les conditions les plus défavorables.

Le test précédent illustre le fait qu'une bande traité physiquement même si elle présente une contrainte de seuil de cisaillement bien inférieure à 2800 Pa, ici 2357 Pa, mais un coefficient de friction dynamique supérieur à 0,25, ici 0,34 permet d'obtenir des résultats d'absence de glissement spire sur spire et de tenue dans le temps identiques à ceux d'une bande qui présente une contrainte de seuil de cisaillement supérieure à 2800 Pa, ici 2937 Pa.

Le traitement qui conduit à un état de surface, caractérisé par la mesure du coefficient de friction dynamique supérieur à 0,25 permet donc bien de s'affranchir de la limite de la valeur de la contrainte de seuil de cisaillement. De plus malgré des conditions de test plus défavorables et une durée supérieure on constate aussi que la bande traitée présente un glissement du trait horizontal non significatif. Elle présente donc un frottement sur la peau amélioré par rapport à la bande non traitée. Ceci est illustré par la valeur du coefficient de frottement dynamique qui est de 0,34, donc supérieur à 0,25, contre 0,22 pour la bande non traitée. Bien que ces valeurs semblent proches ces écarts sont significatifs car on mesure des variations de propriétés faibles. Bien que la présente invention ait été décrite en se référant à des exemples de réalisation spécifiques, il est évident que des modifications et des changements peuvent être effectués sur ces exemples sans sortir de la portée générale de l'invention telle que définie par les revendications. En particulier, des caractéristiques individuelles des différents modes de réalisation illustrés/mentionnés peuvent être combinées dans des modes de réalisation additionnels. Par conséquent, la description et les dessins doivent être considérés dans un sens illustratif plutôt que restrictif.

Il est également évident que toutes les caractéristiques décrites en référence à un procédé sont transposables, seules ou en combinaison, à un dispositif, et inversement, toutes les caractéristiques décrites en référence à un dispositif sont transposables, seules ou en combinaison, à un procédé.

## Revendications

1. Bande de contention qui se présente sous la forme d'un tricot obtenu par la technologie maille jetée, à base de fils synthétiques qui est constitué de 2 surfaces textiles dont la structure textile est identique ou différente reliées entre elles par des fils d'entretoises, chaque surface textile comportant des fils élastiques, et le fil d'entretoise étant un mono filament, ledit tricot présente un allongement longitudinal mesuré selon la norme EN 14704 -1 compris entre 30 et 160%,

   **caractérisée en ce que** la surface textile venant en contact avec la peau présente des fils multi filaments synthétiques, dont les filaments présentent un titre compris entre 1,2 et 5 dtex et le nombre de filaments de chaque fil multi filaments est compris entre 15 et 150, et la surface textile venant en contact avec la peau présente un coefficient de frottement dynamique mesuré selon la norme EN ISO 8295 supérieur ou égal à 0,25 et inférieur ou égal à 1,2,
   dans laquelle la face venant en contact avec la peau présente une quantité de fils thermoplastiques multi filaments de l'ordre de 15 à 40% en pourcentage massique par rapport au poids total du tricot 3D et typiquement de l'ordre de 20 à 25%, et dans laquelle la surface textile venant en contact avec la peau est une surface traitée physiquement avec un traitement physique choisi parmi le grattage, le brossage ou l'émerisage.

2. Bande de contention selon la revendication 1, dans laquelle le fil d'entretoise est un mono filament présentant un titre compris entre 20 et 80 dtex.

3. Bande de contention selon l'une des revendications précédentes, dans laquelle ledit tricot présente une face qui a une structure textile parmi la liste ci-après :

   - charmeuse ;
   - demi-simple à mailles ouvertes ou fermées ;
   - atlas sous un ou plusieurs rangs ;
   - chainette à mailles ouvertes, fermées, ou à alternance de mailles fermées et ouvertes ;

   ladite face étant opposée à la face adaptée pour être mise en contact avec la peau qui a une structure textile qui est un filet présentant une structure textile ajourée.

4. Bande de contention selon l'une des revendications précédentes dans laquelle le tricot présente une épaisseur comprise entre 1 et 2 mm.

5. Bande de contention selon l'une des revendications précédentes, dans laquelle le tricot présente un écart entre les faces compris entre 0,4 et 1,5mm.

6. Bande de contention selon l'une des revendications précédentes dans laquelle le tricot présente un grammage compris entre 160 et 370 g/m$^2$.

7. Bande de contention selon la revendication 6, dans laquelle le tricot présente un grammage compris entre 160 et 300 g/m$^2$.

8. Bande de contention selon l'une des revendications précédentes, dans laquelle le tricot présente un allongement longitudinal tel que défini dans la norme EN 14704-1 compris entre 70 et 100%.

9. Bande de contention selon l'une des revendications précédentes dans laquelle le fil élastique présente un titre compris entre 40 et 80 dtex.

10. Bande de contention selon l'une des revendications précédentes dans laquelle les surfaces textiles comprennent des fils thermoplastiques présentant des titres de 40 à 90 dtex.

11. Bande de contention selon l'une des revendications précédentes dans laquelle la face venant en contact avec la peau présente un coefficient de frottement dynamique mesuré selon la norme EN ISO 8295 supérieur ou égal à 0,25 et inférieur ou égal à 0,5.

12. Bande de contention selon la revendication 1, dans laquelle la surface textile venant en contact avec la peau est une surface émerisée.

**13.** Kit comprenant une ou plusieurs bandes de contention selon l'une des revendications précédentes et un ou plusieurs pansements adaptés pour être disposés sur une plaie préalablement à la bande de contention.

**Patentansprüche**

**1.** Kompressionsbandage, die in Form eines Gestricks vorliegt, das durch die Kettstrick-Technologie auf der Basis von synthetischen Fäden erhalten wird und aus 2 textilen Oberflächen besteht, deren Textilstruktur gleich oder unterschiedlich ist, und die durch Abstandsfäden miteinander verbunden sind, wobei jede textile Oberfläche elastische Fäden umfasst und der Abstandsfaden ein Monofilament ist, wobei das Gestrick eine Längsdehnung gemessen nach der Norm EN 14704 -1 zwischen 30 und 160 % aufweist,

dadurch gekennzeichnet, dass die textile Oberfläche, die mit der Haut in Kontakt gelangt, synthetische Multifilament-Fäden aufweist, deren Filamente einen Titer zwischen 1,2 und 5 dtex aufweisen, und die Anzahl von Filamenten eines jeden Multifilament-Fadens zwischen 15 und 150 beträgt, und die textile Oberfläche, die mit der Haut in Kontakt gelangt, einen dynamischen Reibungskoeffizienten gemessen nach der Norm EN ISO 8295 von mehr als oder gleich 0,25 und weniger als oder gleich 1,2 aufweist, wobei die Fläche, die mit der Haut in Kontakt gelangt, einen Anteil von thermoplastischen Multifilament-Fäden in der Größenordnung von 15 bis 40 Massen-% in Bezug auf das Gesamtgewicht des 3D-Gestricks und in der Regel in der Größenordnung von 20 bis 25 % aufweist, und wobei die textile Oberfläche, die mit der Haut in Kontakt gelangt, eine Oberfläche ist, die physikalisch mit einer physikalischen Behandlung behandelt wurde, die aus Aufrauen, Bürsten und Schleifen ausgewählt ist.

**2.** Kompressionsbandage nach Anspruch 1, wobei der Abstandsfaden ein Monofilament ist, das einen Titer zwischen 20 und 80 dtex aufweist.

**3.** Kompressionsbandage nach einem der vorhergehenden Ansprüche, wobei das Gestrick eine Fläche aufweist, die eine textile Struktur aus der folgenden Liste aufweist:

- Charmeuse,
- Halbeinfach-Gestrick mit offenen oder geschlossenen Maschen,
- Atlas unter einer oder mehreren Reihen,
- Chainette mit offenen, geschlossenen oder abwechselnd geschlossenen und offenen Maschen,

wobei die Fläche, die der Fläche entgegengesetzt ist, die dazu geeignet ist, mit der Haut in Kontakt zu gelangen, die eine Textilstruktur aufweist, die ein Filet ist, eine Ajour-Textilstruktur aufweist.

**4.** Kompressionsbandage nach einem der vorhergehenden Ansprüche, wobei das Gestrick eine Dicke zwischen 1 und 2 mm aufweist.

**5.** Kompressionsbandage nach einem der vorhergehenden Ansprüche, wobei das Gestrick einen Abstand zwischen den Flächen zwischen 0,4 und 1,5 mm aufweist.

**6.** Kompressionsbandage nach einem der vorhergehenden Ansprüche, wobei das Gestrick ein Flächengewicht zwischen 160 und 370 g/m$^2$ aufweist.

**7.** Kompressionsbandage nach Anspruch 6, wobei das Gestrick ein Flächengewicht zwischen 160 und 300 g/m$^2$ aufweist.

**8.** Kompressionsbandage nach einem der vorhergehenden Ansprüche, wobei das Gestrick eine Längsdehnung, wie sie in der Norm EN 14704-1 definiert ist, zwischen 70 und 100 % aufweist.

**9.** Kompressionsbandage nach einem der vorhergehenden Ansprüche, wobei der elastische Faden einen Titer zwischen 40 und 80 dtex aufweist.

**10.** Kompressionsbandage nach einem der vorhergehenden Ansprüche, wobei die textilen Oberflächen thermoplastische Fäden umfassen, die Titer von 40 bis 90 dtex aufweisen.

**11.** Kompressionsbandage nach einem der vorhergehenden Ansprüche, wobei die Fläche, die mit der Haut in Kontakt gelangt, einen dynamischen Reibungskoeffizienten gemessen nach der Norm EN ISO 8295 von mehr als oder gleich 0,25 und weniger als oder gleich 0,5 aufweist.

**12.** Kompressionsbandage nach Anspruch 1, wobei die textile Oberfläche, die mit der Haut in Kontakt gelangt, eine angeschliffene Oberfläche ist.

**13.** Kit, umfassend eine oder mehrere Kompressionsbandagen nach einem der vorhergehenden Ansprüche und einen oder mehrere Verbände, die dazu geeignet sind, vor der Kompressionsbandage auf einer Wunde angeordnet zu werden.

Claims

1. Compression bandage that has the form of a knit obtained by warp stitch technology, based on synthetic yarns which is made up of two textile surfaces whose textile structure is identical or different connected to each other by spacer yarns, each textile surface comprising elastic yarns and the spacer yarn being a monofilament, said knit has a longitudinal elongation measured according to standard EN 14704 -1 of between 30 and 160%, **characterized in that** the textile surface coming into contact with the skin has synthetic multifilament yarns, the filaments of which have a count of between 1.2 and 5 dtex and the number of filaments of each multifilament yarn is between 15 and 150, and the textile surface coming into contact with the skin has a dynamic coefficient of friction measured according to standard EN ISO 8295 greater than or equal to 0.25 and less than or equal to 1.2 wherein the side coming into contact with the skin has a quantity of multifilament thermoplastic yarns of around 15 to 40% by mass percentage relative to the total weight of the 3D knit and typically around 20 to 25%, and the textile surface coming into contact with the skin is a physically-treated surface with a physical treatment chosen among napping, brushing or sueding.

2. Compression bandage according to claim 1, wherein the spacer yarn is a monofilament having a titre comprised between 20 and 80 dtex.

3. Compression bandage according to one of the preceding claims, wherein said knit has a side which has a textile structure among the following list:

   - charmeuse;
   - single cord lap fabric with open or closed loops;
   - atlas under one or more rows;
   - pillar stitch with open or closed loops, or alternating closed and open loops. said side being opposite the side designed to be brought into contact with the skin, which has a textile structure which is a net having an openwork textile structure.

4. Compression bandage according to one of the preceding claims, wherein the knit has a thickness comprised between 1 and 2 mm.

5. Compression bandage according to one of the preceding claims, wherein the knit has a space between the sides comprised between 0.4 and 1.5 mm.

6. Compression bandage according to one of the preceding claims, wherein the knit has a grammage comprised between 160 and 370 g/m$^2$.

7. Compression bandage according to claim 6, wherein the knit has a grammage comprised between 160 and 300 g/m$^2$.

8. Compression bandage according to one of the preceding claims, wherein the knit has a longitudinal elongation such as defined in standard EN 14704-1 comprised between 70 and 100%.

9. Compression bandage according to one of the preceding claims, wherein the elastic yarn has a titre comprised between 40 and 80 dtex.

10. Compression bandage according to one of the preceding claims, wherein the textile surfaces comprise thermoplastic yarns having titres from 40 to 90 dtex.

11. Compression bandage according to one of the preceding claims, wherein the side coming into contact with the skin has a dynamic coefficient of friction measured according to standard EN ISO 8295 greater than or equal to 0.25 and less than or equal to 0.5.

12. Compression bandage according to claim 1, wherein the textile surface coming into contact with the skin is a sueded surface.

13. Kit comprising one or more compression bandages according to one of the preceding claims and one or more wound dressings designed to be positioned on a wound prior to the compression bandage.

[Fig. 1]

[Fig. 2]

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2017089731 A
- EP 1052319 A
- WO 2014033418 A
- WO 2007113340 A